# FASCICULE DE BREVET EUROPEEN

(11) **EP 1 096 953 B1**
(45) Date de publication et mention de la délivrance du brevet: **08.04.2009**
(21) Numéro de dépôt: 99925094.7
(22) Date de dépôt: 15.06.1999
(51) Int. Cl.: A61K 39/12, A61K 39/21, C07K 16/08, C07K 16/10, A61K 39/42

(54) **UTILISATION DE PROTEINES IMMUNOGENES IMMUNOSUPPRESSIVES ET/OU ANGIOGENIQUES RENDUES INACTIVES, PROCEDE DE PREPARATION ET APPLICATIONS PHARMACEUTIQUES OU VACCINALES**
VERWENDUNG VON IMMUNOGENEN IMMUNSUPPRESSIVEN UND/ODER ANGIOGENEN PROTEINEN DIE INAKTIVIERT SIND, VERFAHREN ZUR DEREN HERSTELLUNG, UND VERWENDUNGEN ALS PHARMAZEUTIKUM ODER IMPFSTOFF
USE OF INACTIVATED IMMUNOSUPPRESSIVE AND/OR ANGIOGENIC IMMUNOGENIC PROTEINS, PREPARATION METHOD AND PHARMACEUTICAL OR VACCINE USES

(30) Priorité: 15.07.1998 FR 9809046
(43) Date de publication de la demande: 09.05.2001
(73) Titulaire: NEOVACS, 75016 Paris (FR)
(72) Inventeur: ZAGURY, Jean-François, F-75003 Paris (FR)
(74) Mandataire: Catherine, Alain
(86) Numéro de dépôt international: PCT/FR1999/001423
(87) Numéro de publication internationale: WO 2000/003732

(56) Documents cités:
- WO-A-96/27389
- US-A- 5 308 612
- J. RAPPAPORT ET AL.: "HIV-1 Tat toxoid: Rationale for a Tat vaccine and results of a phase I clinical trial." JOURNAL OF NEUROVIROLOGY, vol. 4, no. 3, avril 1996 (1996-04), pages 97-103, XP002106209 Basingstoke, GB
- CHEMICAL ABSTRACTS, vol. 130, no. 21, 1999 Columbus, Ohio, US; abstract no. 280480, A. GRINGERI ET AL.: "Safety and immunogenicity of HIV-1 Tat toxoid in immunocompromised HIV-1-infected patients." XP002114179 & JOURNAL OF HUMAN VIROLOGY, vol. 1, no. 4, mai 1998 (1998-05), pages 293-298,
- M. BOURSNELL ET AL.: "Construction and characterisation of a recombinant vaccinia virus expressing human papillomavirus proteins for immunotherapy of cervical cancer." VACCINE, vol. 14, no. 16, novembre 1996 (1996-11), pages 1485-1494, XP002106210 Oxford, GB
- Y. TANAKA ET AL.: "Production of a recombinant human T-cell leukemia virus type-I trans-activator (tax1) antigen and its utilization for generation of monoclonal antibodies against various epitopes on the tax1 antigen." INTERNATIONAL JOURNAL OF CANCER, vol. 48, no. 4, 19 juin 1991 (1991-06-19), pages 623-630, XP002106211 Epalinges, Suisse cité dans la demande
- K. SPEIDEL ET AL.: "Priming of cytotoxic T lymphocytes by five heat-aggregated antigens in vivo: conditions, efficiency, and relation to antibody responses." EUROPEAN JOURNAL OF IMMUNOLOGY, vol. 27, no. 9, septembre 1997 (1997-09), pages 2391-2399, XP002106212 Weinheim, Allemagne cité dans la demande
- H. LE BUANEC ET AL.: "A prophylactic and therapeutic AIDS vaccine containing as a component the innocuous Tat toxoid." BIOMEDICINE AND PHARMACOTHERAPY, vol. 52, no. 10, 1998, pages 431-435, XP002106213 Paris, France
- A. GRINGERI ET AL.: "Tat toxoid as a component of a preventive vaccine in seronegative subjects." JOURNAL OF ACQUIRED IMMUNE DEFICICIENCY SYNDROMES AND HUMAN RETROVIROLOGY, vol. 20, no. 4, 1 avril 1999 (1999-04-01), pages 371-375, XP002106214 New York, États-Unis

## Description

La présente invention concerne des composés immunogènes préparés à partir de protéines immunogènes immunosuppressives et/ou angiogéniques rendues inactives, leur procédé de préparation et leurs applications pharmaceutiques ou vaccinales.

On recherche toujours des composés actifs pour lutter contre le cancer qui est le fléau médical majeur de notre époque. De nombreuses thérapies ont été développées avec un succès variable puisqu'il y a encore une mortalité importante. Ces thérapies ont d'abord été l'exérèse chirurgicale pour les tumeurs solides, la radiothérapie et la chimiothérapie. Ces thérapies semblent insuffisantes dans de nombreux cancers pour lesquels aucun succès clinique n'a été enregistré indiquant une prolongation importante de la survie des patients ou leur guérison totale.

Les cancers sont des proliférations de cellules qui peuvent ensuite essaimer dans l'organisme pour former des métastases. Il est connu que le système immunitaire d'un individu normal élimine régulièrement les cellules cancéreuses naissantes (concept d'immunosurveillance), et que la formation d'un cancer est associée 1) à l'échappement du système de surveillance immunitaire local puis à une période avancée du cancer, à une immunosuppression systémique et 2) à une prolifération des cellules endothéliales vasculaires assurant l'apport nutritif des cellules tumorales (néoangiogénèse).

La plupart des agents anticancéreux (chimiothérapie, radiothérapie) utilisés à ce jour luttent contre la réplication des cellules cancéreuses. Ces agents ne visent pas l'environnement particulier nécessaire à la prolifération des cellules cancéreuses, caractérisé à la fois par l'absence d'activité locale (paracrine) des cellules immunitaires antitumorales (échappement immunitaire) et par l'apparition d'une vascularisation intratumorale (néoangiogénèse).

C'est pourquoi, récemment, de nouvelles approches thérapeutiques ont été introduites. Certaines visent à stimuler le système immunitaire antitumeur par thérapie cellulaire ou par l'activation de gènes codant pour des protéines stimulant la réponse immunitaire (thérapie génique) ou encore en immunisant directement contre des antigènes, identifiés comme spécifiques ou associés, de la tumeur du type des M AGE (vaccination). D'autres visent à lutter contre la néoangiogénèse, en utilisant des antimétabolites détruisant les cellules endothéliales (Judah Folkman). Dans ce nouveau contexte il est à noter que De Bruijn et al., (Cancer Res. (1998) 58, pages 724-731) ont décrit l'utilisation de protéines E6 et E7 natives pour induire une réponse cellulaire cytotoxique (CTL) chez des souris pour les protéger contre l'implantation de cellules tumorales.

Or la demanderesse a découvert avec étonnement, après de longues recherches, que des facteurs immunosuppressifs ou angiogéniques à action paracrine sont induits par les tumeurs. Ces facteurs qui sont solubles peuvent, d'une part, empêcher localement les cellules du système immunitaire d'agir efficacement, même si on les stimule (immunosuppression) et, d'autre part, assurer la nutrition des cellules cancéreuses, en activant la prolifération des cellules endothéliales (angiogénèse).

Le phénomène d'échappement aux défenses immunitaires cellulaires de l'hôte par induction de leur paralysie in situ est une stratégie utilisée par de nombreux cancers et est nécessaire à leur survie. Initialement l'immunosuppression reste localisée au niveau de la tumeur, puisque l'individu est encore capable de se défendre contre les autres agressions telles que des infections. Cependant à un stade plus tardif, cette immunosuppression peut s'étendre, se généraliser, ainsi que l'attestent la dissémination de métastases et la grande vulnérabilité du cancéreux face aux infections ceci en dehors même des effets débilitants dus à la chimiothérapie ou radiothérapie. En résumé, cet échappement au contrôle du système immunitaire est dû à une paralysie du système immunitaire (immunosuppression), qui l'empêche de fonctionner normalement. Cette immunosuppression met en jeu des facteurs paralysants, qui sont produits par les cellules cancéreuses ou leur environnement. La paralysie locale des cellules du système immunitaire ou immunosuppression représente donc une arme majeure des cellules cancéreuses qui leur permet d'échapper au système immunitaire de l'hôte. Ainsi, des protéines relâchées par les cellules infectées agissent comme de véritables toxines sur les cellules immunitaires alentour, les dérèglent et bloquent in situ (de manière paracrine) les cellules du système immunitaire, protégeant les cellules infectées.

Les tumeurs malignes sont caractérisées par la présence d'une vascularisation importante, qui assure un afflux de sang nécessaire à la nutrition des cellules cancéreuses. Cette vascularisation est réalisée par l'activation des cellules endothéliales vasculaires induites au contact des cellules tumorales (néoangiogénèse). Les travaux de Judah Folkman ont récemment mis en évidence que le contrôle de la néoangiogénèse tumorale pouvait représenter une arme efficace décisive contre les cancers (Folkman J., Semin. Cancer Biol., 1992, 3 (2) : 65-71).

Dans ce contexte physiopathologique, la demanderesse a découvert, après de longues recherches, dans le cas de l'ATL, du cancer du col utérin, et du sarcome de Kaposi, trois protéines impliquées dans une immunosuppression locale au niveau de la tumeur : il s'agit de la protéine Tax de HTLV 1, de la protéine E7 du papilloma virus, et de la protéine Tat du VIH-1 dans le sarcome de Kaposi. Dans ce dernier cas, il existerait aussi une protéine du HHV8 immunosuppressive, qui expliquerait que le sarcome de Kaposi survient aussi chez des sujets non infectés par le VIH-1. Tat a été impliquée dans le sarcome de Kaposi, mais sans que les chercheurs n'identifient son rôle générateur d'immunosuppression locale qui favorise la génération de sarcomes de Kaposi. De manière intéressante, la demanderesse a trouvé que certaines de ces protéines immunosuppressives, telles la protéine Tat du HIV-1 et la protéine E7 de HPV (souches 16 et 18) ont également des effets activateurs sur les cellules endothéliales vasculaires.

La présente description illustre le blocage, par des anticorps spécifiques, de facteurs solubles relâchés par les cellules tumorales ou infectées par un virus. Par "facteurs solubles", l'on entend des facteurs (en général des protéines) synthétisés par ces cellules et relâchés dans le milieu extracellulaire soit par transport actif soit par diffusion passive. Les facteurs extracellulaires précités peuvent agir in situ soit en inhibant la réponse cellulaire immunitaire, incluant l'activation des lymphocytes T cytolytiques (CTL), soit en perturbant le réseau cytokinique, soit en satisfaisant par la néoangiogénèse les besoins nutritifs de la tumeur. Ces facteurs extracellulaires peuvent être d'origine cellulaire (cytokines) ou, pour les cancers induits par des virus ou les maladies virales, des protéines virales, principalement des protéines de régulation, présentes dans le milieu extracellulaire.

La présente description décrit notamment des moyens permettant d'obtenir, dans le milieu circulant, des anticorps dirigés spécifiquement contre des facteurs délétères, par exemple immunosuppressifs et/ou angiogéniques lesdits anticorps étant susceptibles de bloquer ces facteurs et d'en neutraliser les effets sur les cellules immunitaires et/ou endothéliales. Ces anticorps spécifiques seront soit induits par immunisation active (vaccination) dirigée contre les protéines notamment les facteurs solubles préalablement identifiés, soit administrés passivement (immunisation passive). Les anticorps circulants, présents dans le milieu extracellulaire, en se combinant à ces protéines, pourront en neutraliser les effets indésirables.

La demanderesse a identifié dans au moins trois cancers viro-induits de tels facteurs solubles : le sarcome de Kaposi (HIV-1), le cancer du col de l'utérus (HPV) et la leucémie ATL (HTLV-1 et -2) Ces 3 facteurs, tous d'origine virale, sont respectivement la protéine Tat du HIV-1, la protéine E7 du HPV et la protéine Tax du HTLV -1.

De nombreux cancers sont induits par des virus comme le HIV-1 responsable du sarcome de Kaposi et d'autres cancers, le HPV à l'origine du cancer du col de l'utérus, l'HBV et l'EBV associés, respectivement, à l'Hépatome et à la maladie de Burkitt.

La maladie SIDA (Syndrome d'Immunodéficience Acquise) due au HIV-1 et caractérisée par une immunosuppression cellulaire généralisée, peut se manifester par le sarcome de Kaposi, cancer vasculaire, ou par d'autres formes de cancer dont certaines leucémies ou lymphomes. L'immunosuppression cellulaire observée dans cette maladie qui favorise l'apparition de ces cancers est induite par la protéine Tat de régulation du HIV-1, qui, si elle n'appartient pas à la structure propre du virus, est relâchée par les cellules infectées dans le milieu extracellulaire. Sous cette forme extracellulaire, la protéine Tat, agissant en véritable toxine virale, exerce un effet immunosuppressif sur les cellules immunitaires avoisinantes (Zagury D. et al.; PNAS, 1998, 95 : 3851-56). De plus, au niveau du sarcome de Kaposi, il a été montré que la protéine Tat associée aux cytokines inflammatoires (IFNα, II-1, TNFα) et au BFGF, favorisait la néoangiogénèse qui forme la tumeur (Ensoli B. J. Virol. 1993, 67 : 277-287).

En fait, de par ses propriétés immunosuppressives et angiogéniques, la protéine Tat, présente dans le milieu extracellulaire, favorise dans la maladie Sida, non seulement le développement du sarcome de Kaposi, causé par le virus HHV8, mais également d'autres cancers, incluant des lymphomes ou des leucémies.

Le cancer épithélial du col de l'utérus est provoqué par certaines souches du virus HPV (souches 16 et 18). Les cellules cancéreuses de ce cancer n'expriment que 2 protéines d'apparition précoce de ce virus, la protéine E6 et la protéine E7 qui, toutes deux, ont des effets sur les facteurs régulateurs du cycle cellulaire de la cellule cancéreuse. Par ailleurs, la protéine E7, présente dans le milieu extracellulaire, explique l'apparition chez les malades de faibles taux d'anticorps anti-E7. La protéine E7 extracellulaire, tout comme la protéine Tat extracellulaire peut agir localement comme une toxine sur les cellules stromales (cellules lymphoïdes ou cellules endothéliales) de la tumeur.

Cette protéine E7 a en effet montré expérimentalement des propriétés immunosuppressives et angiogéniques. L'immunosuppression induite par la protéine E7 a été caractérisée par l'inhibition de la prolifération des cellules T stimulées par le PPD ou le toxoïde tétanique, l'inhibition de la prolifération des cellules T stimulées par des cellules allogéniques, la surproduction d'IFNα (cytokine immunosuppressive) par les cellules présentant l'antigène (APC). Le pouvoir angiogénique de la toxine E7 sur les cultures de cellules endothéliales provenant du cordon ombilical de nouveau-nés et prétraitées par la protéine E7 a été suggéré par les observations suivantes : formation de nids cellulaires nombreux, visibles au contraste de phase ; identification par Facs de marquers CAM (ICAM et VCAM) au sein des cellules endothéliales et modification du cytosquelette des cellules en culture observées par immunofluorescence et altération de l'expression du monoxyde d'azote synthase inductible par les cellules endothéliales en culture, en présence de la protéine E7. Comme on le verra dans les exemples de manière plus décisive, le pouvoir angiogénique de la toxine E7 peut être directement démontré " in vitro " par l'activation des cellules endothéliales vasculaires provenant de lignées cellulaires ou de cellules fraîches de cordon ombilical de nouveau-nés induites par la protéine E7 du HPV (souche 16).

L'induction d'anticorps dirigés contre les facteurs viraux extracellulaires telles les protéines Tat, E7 ou Tax, comme des toxines sur les cellules stromales lymphoïdes ou endothéliales intratumorales, implique la préparation d'immunogènes biologiquement dépourvus des effets délétères de la protéine native. De tels immunogènes ou les anticorps spécifiques qu'ils induisent peuvent grâce à leurs propriétés combattre l'immunosuppression et/ou l'angiogénèse présente (s) au sein des tumeurs et donc être utiles en tant que médicaments anticancéreux.

La présente demande décrit l'utilisation :
- d'un produit nouveau obtenu à partir d'une protéine naturelle qui sera modifiée par toute technique comme par voie chimique, physique (dont forme galénique) ou par ingénierie génétique, de telle sorte que ses propriétés immunosuppressives sont inactivées d'au moins 70%, de préférence d'au moins 90%, notamment d'au moins 95%, par un traitement chimique, physique et/ou par construction génétique appropriée.
- d'un anticorps contre une protéine immunopathogène, en particulier immunosuppressive ou angiogénique à action locale induite par une cellule cancéreuse ou une cellule infectée par un virus, pour l'obtention d'un médicament destiné à une utilisation en tant qu'agent anti-immunosuppression locale et/ou en tant qu'agent anti-angiogénique à action locale. Etant donné que des cancers peuvent proliférer grâce à l'immunosuppression locale évoquée ci-dessus et à l'angiogénèse, les produits ci-dessus trouvent en partie leur emploi pour l'obtention d'un médicament destiné à son utilisation en tant qu'anticancéreux.

La protéine naturelle précitée est **caractérisée en ce qu**'elle est une protéine initialement immunosuppressive et/ou angiogénique à action locale induite par des cellules cancéreuses ou par des cellules infectées par un virus ou un fragment de ces protéines.

C'est pourquoi la présente demande décrit l'utilisation d'une protéine provenant de cellules cancéreuses, de cellules infectées par un virus ou de cellules immunitaires ou encore un fragment de cette protéine, **caractérisée en ce que** cette protéine est une protéine initialement immunosuppressive et/ou angiogénique à action locale et en ce que ces propriétés sont rendues inactives d'au moins 70 %, de préférence d'au moins 90%, notamment d'au moins 95%, par un traitement physique et/ou chimique tel qu'une formolation, une carboxamidation, une maleimidation, une oxydation par barbotage d'oxygène, par recombinaison génétique ou encore par un conditionnement adjuvant, ledit traitement lui conservant la propriété d'être reconnue par des anticorps dirigés contre ladite protéine, et lui conservant des propriétés immunogènes suffisantes pour générer des anticorps neutralisant ou bloquant ladite protéine native, pour l'obtention d'un médicament destiné à une utilisation comme agent anti-immunosuppression ou anti-angiogénique locale en tant qu'anticancéreux. Par " agent anti-immunosuppression ou anti-angiogénique " l'on entend que l'agent peut avoir l'un, l'autre ou les deux effets.

Les protéines rendues inactives illustrées dans la présente description, parfois appelées ci-après "protéine inactivées" ou "toxoïdes", permettent de lutter contre les cancers par une approche complémentaire de celles de l'art antérieur et spécifique, visant la paralysie du système immunitaire et/ou encore l'angiogénèse induite(s) par les substances extracellulaires produites localement dans l'environnement des cellules cancéreuses. Cette paralysie immunitaire et/ou l'angiogénèse constituent une véritable barrière protectrice et/ou une source nutritive pour la tumeur.

Les protéines rendues inactives illustrées dans la présente description permettent de lutter en premier lieu contre ces facteurs immunosuppressifs et/ou angiogéniques, par formation d'anticorps contre ces protéines et notamment contre ces facteurs solubles pour permettre au système immunitaire d'agir efficacement et/ou pour bloquer la néoangiogénèse.

Il est important d'utiliser le facteur extracellulaire délétère sous une forme physiquement, chimiquement et/ou génétiquement modifiée (inactivée) et non native (ou naturelle) afin qu'il n'exerce plus ses effets nocifs (paralysie paracrine du système immunitaire ou angiogénèse locale).

Les traitements physiques peuvent être réalisés par la chaleur, les radiations U.V., les rayons X ou le contact avec une atmosphère riche en O₂. Ces traitements physiques générant des modifications intramoléculaires entre radicaux chimiques (groupement thiols par exemple), peuvent de manière appropriée changer la conformation de la molécule, l'inactiver fonctionnellement tout en conservant ses propriétés immunogènes.

Le traitement chimique peut être effectué à l'aide d'un agent de couplage tel qu'un dialdéhyde, ou d'une protéine porteuse activée par un prétraitement à l'aide d'un dialdéhyde, de préférence ou le glutaraldéhyde. Le traitement chimique peut se faire par utilisation d'un monoaldéhyde, notamment de formaldéhyde. On peut à cet égard se reporter à l'enseignement de WO-A-96/27389.

Le traitement chimique peut se faire notamment par d'autres procédés telle la carboxyméthylation. Un exemple de technique de carboxyméthylation est illustré ci-après dans la partie expérimentale. Le traitement chimique peut se faire également par N éthylmaléidation associée ou non à une glutaraldéhydation.

L'immunogène peut être inactivé grâce à une présentation galénique au sein d'un liquide huileux, tel l'adjuvant incomplet de Freund ou encore susceptible de modifier les liaisons non covalentes (forces électrostatiques, forces de Van der Waals ou liaisons hydrogène) nécessaires à ses effets toxiques.

Les modifications génétiques peuvent être obtenues par ingénierie génétique opérant des insertions, des délétions ou des substitutions de résidus, opérations destinées à diminuer ou supprimer les sites fonctionnels délétères de la molécule naturelle. Les mutants génétiques pourront ou non subir un traitement chimique et/ou physique complémentaire. Les protéines modifiées ci-dessus peuvent par exemple être préparées à partir d'une protéine ayant une séquence identique ou similaire à une séquence peptidique d'une protéine immunopathogène, en particulier immunosuppressive ou angiogénique, telle que la protéine Tat du VIH-1, la protéine E7 du papilloma virus ou la protéine Tax de HTLV1 ou d'un fragment de ces protéines et être obtenues par exemple par synthèse peptidique conventionnelle sur résine ou par génie génétique. Tous ces procédés sont bien connus de l'état de la technique.

Afin de vérifier que la protéine native immunosuppressive et/ou angiogénique est bien reconnue par des anticorps dirigés contre ladite protéine immunosuppressive modifiée ou son fragment modifié selon l'invention, on peut par exemple vérifier immunologiquement par Elisa en présence d'anticorps spécifiques, la formation de complexes antigène-anticorps comme on le verra ci-après dans la partie expérimentale.

Dans des conditions préférentielles de mise en oeuvre, le composé immunogène provient d'un composé natif (protéine ou fragment polypeptidique) carboxamidé ou maléimidé.

Afin de déterminer si les propriétés immunogènes de la protéine modifiée immunosuppressive et/ou angiogénique ou un fragment de cette protéine ont été suffisamment conservées (c'est à dire si il ou elle a été inactivé(e) mais pas dénaturé) pour créer des anticorps bloquant les effets de ladite protéine native, on peut par exemple immuniser des mammifères (lapins, rats, souris) à l'aide d'un composé immunogène selon l'invention et vérifier que les anticorps produits neutralisent les activités immunosuppressives ou angiogénique de la protéine, comme on le verra pour la protéine Tat du VIH-1, la protéine E7 du papilloma virus et la protéine Tax de HTLV1 dans la partie expérimentale.

Afin de déterminer si la protéine immunosuppressive modifiée ou fragment a perdu au moins la proportion désirée de ses propriétés immunosuppressives, on peut par exemple étudier l'effet de la protéine immunosuppressive sur l'immunosuppression des cellules mononucléées du sang périphérique humain (PBMC).

La protéine immunosuppressive ou angiogénique modifiée et immunogène peut dériver d'une quelconque des protéines notamment immunosuppressives à action locale induites par les tumeurs ; on retient particulièrement la protéine Tat du virus VIH-1, la protéine E7 du papilloma virus ou la protéine Tax du virus HTLV1. On retient aussi la lectine mannane-dépendante produite par des cellules immunitaires activées.

Par "dérivent" ou "dériver" d'une protéine immunopathogène, en particulier immunosuppressive ou angiogénique à action locale produite par des cellules cancéreuses ou infectées par un virus ou produite par des cellules immunitaires, l'on entend que le composé immunogène peut être constitué de la totalité ou d'un fragment de la protéine immunopathogène, en particulier immunosuppressive ou angiogénique de départ.

Il peut comporter une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés; les modifications doivent généralement concerner moins de 40% d'acides aminés, de préférence moins de 20% et tout particulièrement moins de 10% de la protéine immunopathogène, en particulier immunosuppressive ou angiogénique. Il est important que la protéine ou fragment modifié ne soit pas dénaturé comme on peut le faire par exemple par un traitement physique comme la chaleur afin de préserver ses sites conformationnels pour que les anticorps induits par les dérivés modifiés soient actifs vis à vis de la protéine native.

Dans des conditions préférentielles, les composés immunogènes de l'invention comportent au moins 50% de la totalité ou d'un segment de la protéine immunopathogène, en particulier immunosuppressive ou angiogénique, de préférence au moins 70%, particulièrement au moins 90%, et tout particulièrement la totalité ou quasi-totalité de ladite protéine immunosuppressive ou de la protéine angiogénique.

De manière générale, en ce qui concerne les modifications, l'homologie ou la similitude entre l'immunogène modifié et la protéine ou partie de protéine immunosuppressive native, ainsi que les dimensions du composé immunogène, de même que les modalités d'utilisation, ou de couplage du composé immunogène selon l'invention à une protéine immunogène telle que le toxoïde tétanique, on peut en particulier se référer à WO-A-86/06 414 ou à EP-A-0.220.273 ou encore à WO-A-96/06 414.

La présente demande décrit également un composé immunogène **caractérisé en ce qu**'il est une protéine initialement immunosuppressive et/ou angiogénique à action locale provenant de cellules cancéreuses, de cellules infectées par un virus ou de cellules immunitaires ou encore un fragment de cette protéine et en ce que ses propriétés immunosuppressives et/ou angiogéniques sont inactivées d'au moins 70 % par un traitement physique et/ou chimique, par recombinaison génétique ou encore par un conditionnement adjuvant, ledit traitement lui conservant la propriété d'être reconnue par des anticorps dirigés contre ladite protéine, et lui conservant des propriétés immunogènes suffisantes pour générer des anticorps neutralisant ou bloquant ladite protéine native, à l'exception de Tat de VIH1 carboxyméthylé ou d'un fragment de cette protéine.

Parmi les composés immunogènes décrits ci-dessus, on préfère les composés immunogènes caractérisés en ce qu'il dérivent d'une des protéines immunosuppressives et/ou angiogéniques suivantes :
- protéine Tax d'un virus HTLV-1
- protéine E7 du papilloma virus
- lectine mannane-dépendante produite par des cellules immunitaires et particulièrement les lymphocytes T activés.

On préfère aussi un composé immunogène tel que défini ci-dessus qui est un produit obtenu par recombinaison génétique présentant une homologie peptidique de 70% au moins avec les protéines Tat du HIV-1, Tax du HTLV1 ou 2 et E7 de l'HPV ou la lectine mannane-dépendante produite par des cellules immunitaires activées ou avec un segment de ces protéines.

On préfère également un composé immunogène tel que défini ci-dessus **caractérisé en ce qu**'il est carboxamidé ou qu'il est maléimidé.

On préfère enfin un composé immunogène tel que défini ci-dessus caractérisé par un conditionnement adjuvant qui le rend biologiquement inactif, telle qu'une émulsion huileuse en adjuvant de Freund incomplet (IFA).

On peut aussi faire dériver d'un mutant homologue le composé immunogène désiré.

L'invention a pour objet un composé immunogène **caractérisé en ce qu**'il est une protéine initialement immunosuppressive et/ou angiogénique choisie parmi :
- la protéine Tat du virus HIV-1 ;
- la protéine Tax d'un virus HTVL-1 ; et
- la protéine E7 du Papillomavirus ;
ou encore un fragment comprenant au moins 50% de la totalité de cette protéine, et en ce que cette protéine est inactivée par traitement chimique par carboxamidation ou maléimidation.

La présente description illustre également un procédé de préparation d'un composé immunogène tel que défini ci-dessus, **caractérisé en ce que** l'on soumet une protéine initialement immunosuppressive ou angiogénique à action locale induite par une tumeur cancéreuse, ou une protéine mutante homologue, ou un fragment de cette protéine, à un traitement physique, chimique, ou à une recombinaison génétique, sélectionne puis purifie le composé attendu, ou incorpore une protéine native ou inactivée dans un conditionnement adjuvant. En effet, par un conditionnement galénique d'une protéine active physiologiquement, on peut masquer son activité biologique tout en conservant son immunogénicité.

La présente description illustre notamment un procédé de préparation d'un composé immunogène tel que défini ci-dessus, **caractérisé en ce que** l'on effectue un traitement chimique qui comprend un traitement à l'aide d'un aldéhyde suivi d'un couplage à une protéine porteuse, ainsi qu'un procédé **caractérisé en ce que** l'on effectue un traitement chimique qui comprend un traitement bloquant les résidus SH par carboxamidation, carboxyméthylation ou maléimidation.

La réaction de carboxyméthylation permet de modifier les groupements thiols (groupements sulfhydryl) présents au niveau des résidus cystéines de l'enchaînement en acides aminés. La carboxyméthylation inactive certaines fonctions toxiques dépendantes des groupements SH comme l'a rapporté pour la protéine Tat Frankel et al. Cell Vol 55 (1988). Ces groupements réagissent avec l'acide iodoacétique ou l'iodoacétamide par une réaction de S-carboxyméthylation ou S-carboxyamidométhylation, respectivement.

La réaction de maléimidation bloque également les groupements SH pour former des complexes S-maléimides.

Par exemple, la protéine Tat possède 7 cystéines. Ces cystéines participent à la formation de ponts disulfure inter et intra-chaînes et contribuent à la formation d'oligomères.

Le produit de la réaction est dans chaque cas un résidu S-carboxyméthylcystéinyl ou S-carboxyméthylamidocystéinyl.

Un fragment peut comporter de 8 à 110 acides aminés par exemple, de préférence de 12 à 60 acides aminés et notamment de 12 à 40 acides aminés. Un tel fragment peut comporter aussi du ou des côtés C ou N terminal de 1 à 5 acides aminés supplémentaires c'est-à-dire différents du segment d'origine. Un fragment doit en outre comporter une cystéine au moins pour pouvoir faire l'objet de la carboxyméthylation.

La réaction de carboxyméthylation peut aussi être réalisée avec d'autres agents chimiques comme l'acide performique, l'acide 3-bromopropionique, l'éthylénéimine, le bromure de (2-bromoéthyl) triméthylammonium, le 2-bromoéthane sulfonate, la 1,3-propanesulfone etc....

Dans des conditions préférentielles de mise en oeuvre du procédé ci-dessus décrit, ladite protéine ou ledit fragment de départ peut se présenter sous forme fusionnée à un marqueur (FP) ou non fusionnée (P). La forme FP peut modifier per se la conformation moléculaire et par là modifier son activité.

Les protéines ou fragments de départ du procédé sont des produits connus dont des procédés d'inactivation peuvent avoir été décrits par hasard dans la littérature par exemple pour une étude structurelle mais en aucun cas utilisés comme vaccin, par exemple pour Tat de VIH-1 par Frankel A.D. et al. (Cellular uptake of the tat protein from human immunodeficiency virus, Cell, 1988, 55 : 1189-93), pour le Tat par Tanaka et al. Int. J. Cancer, 1991, vol 48, pages 623-630 ou n'ont pas encore été décrits comme l'inactivation de la protéine E7 décrite par Speidel et al, Eur. J. Immunol., 1997, 27, pages 2391-2399. Ces protéines de départ peuvent être même commercialisées (Immunodiagnostics Inc., Cat # 1002-2) ou peuvent être préparées de manière conventionnelle.

On peut en particulier préparer les protéines ou fragments de départ ci-dessus par :
1) Synthèse par génie génétique ou par synthèse biochimique ;
2) Purification

Par génie génétique, on peut purifier les protéines produites par chromatographie d'affinité en utilisant par exemple des anticorps dirigés contre la protéine ou un de ses fragments ; on peut aussi synthétiser la protéine fusionnée à un marqueur (FP) qui servira à l'accrochage à une colonne d'affinité.

Dans d'autres conditions préférentielles de mise en oeuvre du procédé ci-dessus décrit, lorsque la protéine ou fragment est fusionné à un marqueur (FP), il est soumis à :
- une concentration, par exemple, par ultrafiltration ;
- un désalage, par exemple, par gel filtration ;
- un traitement au bromure de cyanogène ou l'entérokinase pour cliver la protéine de fusion et libérer ainsi la protéine ou fragment ;
- une concentration et diafiltration ;
- une chromatographie par échange cationique ;
- une concentration par ultrafiltration, suivie par une filtration sur gel d'exclusion.

La réaction au bromure de cyanogène ci-dessus permet de cliver les thioéthers. L'action du bromure de cyanogène sur les molécules polypeptidiques est sélective en effectuant un clivage au niveau des résidus méthionine existants. Cette réaction aboutit à la formation de 2 fragments polypeptides par résidu méthionine. Cette réaction peut être avantageusement couplée avec la réaction de carboxyméthylation précédemment décrite mais elle n'est pas nécessaire à l'inactivation.

Dans d'autres conditions préférentielles de mise en oeuvre du procédé ci-dessus décrit, on prépare la protéine ou le fragment attendu(e) couplé(e) à un composé permettant sa purification, par exemple à un fragment peptidique contenant plusieurs histidines, de préférence en séquence continue de 4, 5, notamment 6 histidines ou plus permettant la fixation à une colonne de Nickel. Dans la mesure où la présence de ce composé n'induit pas de toxicité et ne modifie pas défavorablement l'immunogénicité de la protéine ou de fragment, il n'est pas nécessaire de le cliver après purification. Toutefois, dans des conditions de réalisation préférée, on clive ce composé pour l'éliminer.

Les protéines modifiées qui sont des protéines initialement notamment immunosuppressives et/ou angiogéniques à action locale induites par les tumeurs cancéreuses et fragments et dont les propriétés immunosuppressives sont inactivées par un traitement approprié, illustrées dans la présente description, possèdent de très intéressantes propriétés pharmacologiques. Elles sont douées notamment de remarquables propriétés antagonistes des propriétés des protéines immunosuppressives et/ou angiogéniques à action locale induites par une tumeur cancéreuse.

Ces propriétés sont illustrées ci-après dans la partie expérimentale. Elles justifient l'utilisation des protéines modifiées ci-dessus décrites à titre de médicament.

C'est pourquoi l'invention a encore pour objet des médicaments **caractérisés en ce qu**'ils sont constitués des composés immunogènes tels que définis par les revendications, pour leur utilisation dans une méthode de traitement thérapeutique du corps humain ou animal, ainsi que l'utilisation d'un tel composé immunogène pour la préparation d'un médicament curatif ou préventif destiné à la neutralisation des effets immunosuppressifs et/ou angiogéniques des protéines immunosuppressives et/ou angiogéniques ci-dessus, et notamment de la protéine Tat du VIH-1, la protéine E7 du papilloma virus et la protéine Tax de HTLV1. En effet, les composés selon l'invention ont perdu leurs propriétés immunosuppressives ou leurs propriétés angiogéniques et peuvent donc être administrés chez l'homme comme on le verra ci-après dans la partie expérimentale.

Les médicaments selon la présente invention trouvent leur emploi par exemple dans le traitement tant curatif que des cancers, notamment des cancers induits par des virus comme, par exemple, l'ATL (Acute T cell leukemia) causé par le HTLV 1, ou le cancer du col utérin causé par le papilloma virus, ou encore le lymphome de Burkitt ou le sarcome de Kaposi causés par des virus de la famille herpès, respectivement l'Epstein-Barr (EBV) et le HHV8.

Les composés immunogènes selon l'invention peuvent être utilisés comme suit :

On administre à un patient un composé immunogène selon la présente invention, par exemple par voie sous-cutanée ou intramusculaire, en quantité suffisante pour être efficace sur le plan thérapeutique, à un sujet ayant besoin d'un tel traitement. La dose administrée peut aller par exemple de 10 à 1000 µg par voie sous-cutanée, une fois par mois pendant trois mois, puis périodiquement en fonction du taux des anticorps sériques induits, par exemple tous les 2-6 mois. On pourra administrer dans une même préparation deux ou plusieurs molécules immunogènes différentes pour induire des anticorps neutralisant tous les sites fonctionnels délétères au cas où une seule molécule immunogène ne porte pas tous les sites actifs de la toxine ou de la cytokine surproduite que l'on veut neutraliser.

L'invention a aussi pour objet les compositions pharmaceutiques qui renferment au moins un composé immunogène tel que défini par les revendications à titre de principe actif.

A titre de médicaments, les composés immunogènes de l'invention peuvent être incorporés dans des compositions pharmaceutiques destinées à n'importe quelle voie conventionnelle en usage dans le domaine des vaccins, en particulier la voie sous-cutanée, la voie intramusculaire, la voie intraveineuse ou la voie orale. L'administration peut avoir lieu en dose unique ou répétée une ou plusieurs fois après un certain intervalle de temps.

C'est pourquoi la présente demande a également pour objet une composition pharmaceutique, curative ou préventive, caractérisée en ce qu'elle comprend à titre de principe actif, un ou plusieurs composés immunogènes nouveaux tels que définis par les revendications. Le composé immunogène peut être conditionné seul ou mélangé à un excipient ou mélange d'excipients pharmaceutiquement acceptables tel qu'un adjuvant.

La présente demande décrit un vaccin contenant à titre d'immunogène, un composé immunogène défini ci-dessus et notamment une protéine initialement immunosuppressive et/ou angiogénique à action locale induite par une tumeur cancéreuse ou un fragment de cette protéine dont les propriétés immunosuppressives et/ou angiogéniques sont inactivées d'au moins 70 % par un traitement approprié.

La présente invention a encore pour objet un procédé de préparation d'une composition ci-dessus décrite, **caractérisé en ce que** l'on mélange, selon des méthodes connues en elles mêmes, le ou les principes actifs avec des excipients acceptables, notamment pharmaceutiquement acceptables.

L'administration à un patient de composés immunogènes selon l'invention correspond à une immunothérapie active. Il peut être intéressant également de procéder à une immunothérapie passive, c'est à dire de fournir à un patient directement des anticorps dont il a besoin pour neutraliser les effets nocifs des protéines ci-dessus par exemple des protéines immunosuppressives à action locale induites par des tumeurs.

Ces anticorps par exemple anti-protéines immunosuppressives et/ou angiogéniques peuvent être obtenus classiquement et à titre d'exemple après immunisation d'un mammifère, homme ou animal, à l'aide d'un composé immunogène tel que défini ci-dessus, par clonage de lymphocytes B humains transformés par le virus Epstein-Barr puis récupération des anticorps attendus sécrétés par lesdits lymphocytes B transformés, ou encore par recombinaison génétique à partir d'une librairie de phages.

C'est pourquoi la présente demande illustre également de tels procédés de préparation d'anticorps anti-protéines notamment immunosuppressives et/ou angiogéniques d'une tumeur cancéreuse et en particulier d'anticorps anti-protéine E7 du papilloma virus ou anti-protéine Tax de HTLV1, et notamment un procédé de préparation d'un anticorps ci-dessus **caractérisé en ce que** l'on immunise un mammifère à l'aide d'un composé immunogène tel que défini ci-dessus, puis récupère les anticorps formés.

La présente demande décrit aussi un anticorps anti-protéine immunosuppressive ou angiogénique d'une tumeur cancéreuse et en particulier des anticorps polyclonaux ou monoclonaux obtenus à partir de mammifères immunisés avec un composé immunogène défini ci-dessus et notamment une protéine immunosuppressive ou angiogénique d'une tumeur cancéreuse biologiquement inactivée mais immunogène, notamment la protéine E7 du papilloma virus ou la protéine Tax de HTLV1, ou leurs fragments. Ces anticorps administrés passivement, qu'ils soient allogéniques (humains) ou xénogéniques (animaux), pourront être des anticorps monoclonaux ou polyclonaux complets ou des fragments F(ab')2 ou Fab de l'anticorps.

Par "anticorps anti-protéine immunosuppressive ou angiogénique d'une tumeur cancéreuse", l'on entend des anticorps monoclonaux ou polyclonaux ou des fragments F(ab')2 ou Fab de ces anticorps ou encore des anticorps anti-protéine immunosuppressive ou angiogénique d'une tumeur cancéreuse obtenus par construction génétique à partir d'une librairie de phages.

Les anticorps xénogéniques proviennent d'animaux hyperimmunisés avec un composé immunogène selon l'invention, notamment avec la protéine E7 du papilloma virus ou la protéine Tax de HTLV1 ou ses dérivés (fragments peptidiques de la protéine E7 du papilloma virus ou de la protéine Tax de HTLV1 détoxifiés selon l'invention), et sont
- soit polyclonaux provenant d'animaux hyperimmunisés,
- soit monoclonaux, obtenus après hybridation selon la technique que Kohler et Milstein de cellules spléniques ou d'adénocytes avec une lignée myélomateuse, type x63, notamment x63AG3. Dans ce cas on préfère les anticorps équins ou de lapin.

La présente demande décrit aussi un procédé de préparation d'anticorps anti-protéine immunosuppressive ou angiogénique d'une tumeur cancéreuse, **caractérisé en ce que** l'on immunise un mammifère, homme ou animal, avec un composé immunogène tel que défini ci-dessus.

La présente demande décrit aussi un procédé de préparation d'anticorps monoclonaux anti-protéine immunosuppressive ou angiogénique d'une tumeur cancéreuse, **caractérisé en ce que** l'on utilise des cellules B provenant d'individus immunisés par un composé immunogène selon la présente invention, lesdites cellules B étant transformées par le virus EBV et produisant des anticorps spécifiques anti-protéine immunosuppressive ou angiogénique d'une tumeur cancéreuse.

Les cellules EBV + ci-dessus peuvent être cultivées de manière à produire les anticorps attendus. Ces cellules, comme on l'a vu, proviennent notamment de malades immunisés par une protéine immunosuppressive ou angiogénique native d'une tumeur cancéreuse, ou par un composé immunogène selon l'invention.

La présente demande décrit aussi un procédé de préparation d'anticorps selon l'invention monoclonaux, dirigés contre une protéine immunosuppressive ou angiogénique d'une tumeur cancéreuse, effectivement immunosuppressive ou non, **caractérisé en ce que** l'on prépare des hybridomes de mammifère, notamment de souris à partir de splénocytes ou adénocytes notamment de souris immunisées par une protéine immunosuppressive ou angiogénique native ou un composé immunogène selon l'invention, et de cellules de myélome, de préférence de lignée x63, selon les procédés bien connus de l'état de la technique (Kohler et Milstein).

La présente demande décrit aussi un procédé d'obtention d'anticorps anti-protéine immunosuppressive ou angiogénique d'une tumeur cancéreuse, par la technologie de recombinaison génétique, **caractérisé en ce que** l'on utilise à titre d'immunogène un composé immunogène tel que défini ci-dessus.

La présente demande décrit également des fragments F(ab')2 ou Fab desdits anticorps ; ceux-ci peuvent être obtenus par digestion enzymatique par exemple.

La présente demande décrit aussi un procédé d'immunisation passive de sujets cancéreux, utilisant des anticorps spécifiques anti-protéine immunosuppressive ou angiogénique d'une tumeur cancéreuse et spécialement anti-protéine E7 du papilloma virus ou anti-protéine Tax de HTLV1 neutralisant ou bloquant les effets nocifs de cette protéine et pouvant être préparés comme indiqué ci-dessus, ou des fragments F(ab')2 ou F(ab) de ces anticorps.

La présente demande décrit également un procédé d'immunisation active **caractérisé en ce que** l'on utilise à titre d'immunogène un composé immunogène tel que défini ci-dessus avantageusement associé à un adjuvant d'immunité minéral, huileux ou de synthèse, ou encore un composé immunogène tel que défini ci-dessus, avantageusement couplé par exemple à l'aide d'un dialdéhyde ou associé à une protéine augmentant son immunogénicité.

Ces immunisations peuvent être réalisées tant à titre curatif qu'à titre préventif.

A titre d'immunogènes, pour tous les procédés ci-dessus et ci-après, on utilise de préférence un dérivé de la protéine E7 du papilloma virus ou de la protéine Tax de HTLV1.

La description illustre aussi une composition pharmaceutique comprenant, à titre de principe actif curatif ou préventif , au moins un anticorps anti-protéine immunosuppressive ou angiogénique d'une tumeur cancéreuse tel que défini ci-dessus ou obtenu selon les procédés ci-dessus.

La description illustre aussi l'utilisation d'un composé immunogène ou d'un anticorps ci-dessus pour la préparation d'un médicament destiné au traitement des effets immunosuppressifs locaux d'une protéine à action immunosuppressive locale ou angiogénique d'une tumeur cancéreuse.

En résumé et notamment, la présente demande décrit l'usage à titre préventif, ou curatif chez le sujet cancéreux d'anticorps spécifiques de manière à bloquer l'action notamment immunosuppressive d'une protéine à action immunosuppressive locale ou angiogénique d'une tumeur cancéreuse et notamment de la protéine Tat du VIH-1, de la protéine E7 du papilloma virus ou de la protéine Tax de HTLV. Ces anticorps spécifiques pourront provenir :
1. du sujet lui-même, induits par une immunisation active (vaccination) avec une protéine immunosuppressive locale d'une tumeur cancéreuse privée desdits effets immunosuppressifs mais immunogène ( on a préservé les propriétés susceptibles d'induire la formation des anticorps, lorsque la protéine est présentée et préparée de manière appropriée, couplée ou non à un "carrier", agrégée ou non, en présence ou non d'adjuvant) ou
2. d'un organisme étranger, allo- ou xénogénique, administré au sujet par immunisation passive (sérothérapie). Ces anticorps administrés passivement, pourront être des anticorps monoclonaux ou polyclonaux complets ou des fragments F(ab')2 ou Fab de l'anticorps.

La demande décrit aussi des compositions pharmaceutiques.
a) Une composition pharmaceutique comprenant à titre d'agent préventif ou curatif une protéine initialement immunosuppressive ou angiogénique locale d'une tumeur cancéreuse mais privée desdits effets immunosuppressifs et immunogène selon l'invention.
b) Une composition pharmaceutique comprenant à titre d'agent préventif ou curatif des anticorps anti-protéine à action immunosuppressive ou angiogénique locale d'une tumeur cancéreuse produits à partir d'organismes immunisés contre ladite protéine ou ses fragments F(ab')2 ou Fab, selon l'invention.

En outre la demande décrit également un kit comprenant une composition pharmaceutique vaccinale qui en plus du principe actif (protéine initialement immunosuppressive ou angiogénique locale d'une tumeur cancéreuse mais privée desdits effets immunosuppressifs ou angiogéniques et immunogène ou ses dérivés ou anticorps anti-protéine immunosuppressive ou angiogénique locale d'une tumeur cancéreuse) peut comprendre un adjuvant et/ou un autre immunogène a propriétés anticancéreuses.

Enfin l'invention illustre une composition pharmaceutique sous une forme galénique conventionnelle. En particulier on associe le principe actif selon l'invention en quantité suffisante pour être efficace d'un point de vue thérapeutique avec un diluant ou un support acceptable d'un point de vue pharmaceutique.

Pour diminuer la charge immunosuppressive produite par la tumeur et améliorer encore mieux la réponse immunitaire, on pourra associer à cette immunisation anti-suppressive des moyens plus classiques visant à diminuer la taille de la tumeur comme la chimiothérapie, la radiothérapie, l'exérèse chirurgicale ou encore l'adjonction de gènes suppresseurs des tumeurs apportés par des techniques de la thérapie génique (ADN véhiculés par des vecteurs viraux, vecteurs lipidiques etc..) ou encore comme l'immunisation active contre des protéines sans action immunosuppressive ou angiogénique locale comme les MAGE ou des protéines de structure comme celles du papilloma virus.

On pourra aussi associer d'autres immunisations anti-suppressives et/ou angiogéniques par exemple en immunisant contre des cytokines ou des lectines susceptibles de servir de médiateur à l'action suppressive sur les cellules immunitaires ou associer des immunisations contre des antigènes tumoraux classiques (non immunosuppressifs ou angiogéniques) susceptibles d'augmenter la réponse immunitaire cellulaire particulièrement tueuse (cellules CTL ou cellules NK) dirigée contre les cellules tumorales ou infectées par des virus. L'avantage de ces associations est qu'elles permettront au système immunitaire de mieux répondre à l'immunisation anti-immunosuppressive et par conséquent de mieux se reconstituer.

Pour résumer, à l'immunisation contre un facteur immunosuppressif ou angiogénique paracrine présenté sous une forme inactive mais toujours immunogène on pourra associer des méthodes plus classiques comme radiothérapie, chimiothérapie exérèse chirurgicale ou traitement par gènes suppresseurs ou encore des immunisations contre des cytokines ou des lectines produites par des cellules immunitaires (cellules T ou APC) médiant l'action immunosuppressive et/ou angiogénique ou contre des antigènes tumoraux.

En effet, dans certains cancers, même d'origine virale, des facteurs solubles d'origine cellulaire, telles les cytokines ou les lectines peuvent également jouer localement un rôle de médiateur de l'immunosuppression et/ou de l'angiogénèse au sein des tumeurs. C'est le cas de l'IFNα, cytokine immunosuppressive, surproduit localement au sein des tissus lymphoïdes infectés par le HIV dans la maladie Sida.

L'expérimentation in vitro sur des cellules mononucléées du sang (PBMC) infectées par le HIV-1 a montré que la protéine Tat était impliquée dans la surproduction par les APC de la cytokine IFNα immunosuppressive. De manière intéressante, la protéine E7 du HPV, tout comme la protéine Tat du HIV-1, induit la surproduction d'IFNα immunosuppressif par les APC.

En conséquence, on pourra également combattre l'échappement immunitaire et/ou l'angiogénèse des cancers, en induisant ou en administrant des anticorps dirigés spécifiquement contre les cytokines dont la surproduction est responsable d'immunopathogénèse, en particulier d'immunosuppression, tel l'IFNα et/ou de l'angiogénèse, tel le TNFα conformément à la demande de brevet internationale WO 92/22 577.

Ainsi les effets immunosuppressifs dus à la surproduction d'IFNα dans les cancers de la maladie Sida et du col utérin tout comme ceux dus à la surproduction de TGFB dans les gliomes viro-induits pourront être bloqués par des anticorps dirigés contre ces cytokines naturelles induits par une immunisation active (vaccination) utilisant des cytokinoïdes (cytokines modifiées biologiquement inactives mais immunogènes) comme vaccin. De tels anticorps pourront également être administrés passivement (immunisation passive).
La figure 1 représente les réponses anti-Tat des 5 sujets immunisés dans l'expérimentation 5 ci-après, déterminées par ELISA sur plaques cotées par la protéine Tat native, après l'immunisation au Tat toxoïde chez les 5 sujets et les différents rappels.
La figure 2 représente les titres en anticorps obtenus après toutes les immunisations, et ceci comparé à 2 contrôles non immunisés.
La figure 3 représente l'induction de l'expression de NOsynthase inductible (iNOS) dans les cellules endothéliales vasculaires humaines (HUVEC) prétraitées par la protéine HPV₁₆E7 et cultivées en présence des cytokines TNFα, IL1 et IFNγ.
La figure 4 représente l'induction d'ARN messager de l'iNOS après 6 heures de traitement par la protéine E7 (100 ng/mml) et les cytokines : TNFα (5 ng/ml), IL1-β (1 ng/ml) et IFNγ (1000 U/ml).
La figure 5 représente les résultats de cytométrie de flux de cellules endothéliales humaines (HUVEC) prétraitées ou non par la protéine oncogène E7 et cultivées pendant 12 heures en présence des cytokines IFNγ, TNFα et IL1 (effet de la protéine E7 sur l'expression de la molécule d'adhésion 1 ICAM-1 dans les cellules endothéliales du cordon ombilical humain).

Les exemples et expérimentations qui suivent illustrent la présente demande.

### Exemple 1. Fabrication de Protéine Tat inactivée par traitement chimique. Préparation

On cultive 2 ml de la bactérie déposée à la Collection Nationale de Cultures de Micro-organismes de Paris le 26 Décembre 1997 sous le N° I 1964 qui est une bactérie E coli dans laquelle on a inséré par transfection un plasmide recombinant contenant un gène codant pour un polypeptide comportant un fragment constitué de 6 histidines, associé au gène de la protéine Tat, dans 400 ml d'un milieu de culture de base (extrait de levure 5g/l, tryptone 1g/l, chlorure de sodium 1g/l) et 40 ml d'une solution 1 (CaCl₂2H₂O : 0,175 g/l, MgSO₄7H₂O : 5,9 g/l, glucose : 6 g/l). On incube ainsi la culture à 30°C pendant 10 heures à 250 tr/mn.

La préfermentation ci-dessus est suivie d'une fermentation dans des conditions analogues, en maintenant le niveau d'oxygène dissous à un niveau de 70 % de la saturation par régulation de l'agitation. Lorsque la densité optique mesurée à 650 nm atteint la valeur de 6, on ajoute 100 ml d'une solution stérile à 3,75 % d'IPTG (isopropyle β-d-thiogalactopyranoside) dans de l'eau désionisée. En même temps, on supplémente le milieu avec une solution stérile d'extrait de levure à 25 %. On ajoute alors une solution comprenant MgSO₄7H₂O : 8,5 g/l, glucose : 300 g/l, (NH₄)₂SO₄ : 106 g/l, oligo-éléments, en maintenant la concentration de glucose au delà de 2 g/l. On récolte alors la masse bactérienne 4 heures après l'introduction de l'IPTG. On procède à une diafiltration contre un tampon tris 0,1 M, NaH₂PO₄ 0,1 M, ditriothéitol 1 Mm, pH 8,0, par filtration à contre-courant (seuil 0,3 µm).

Trois litres du produit brut ci-dessus sont lysés par 4 passages à une suppression de 500 bars. Le lysat est alors centrifugé pendant 15 mn à 4°C et 5000 tr/mn. On ajoute alors au surnageant une quantité suffisante d'urée pour obtenir une solution 8 M et on ajuste le pH à 8.

On procède alors à une purification par chromatographie d'affinité sur une colonne nickel-NTA agarose (Qiagen) préalablement équilibrée avec 600 ml de tampon A (urée 8M, NaH₂PO₄ 0,1 M, tris-HCl 0,1 M, DTT 1 Mm, pH 8,0). Une fois chargée, la colonne a été lavée avec 250 ml de tampon A. On élue alors la protéine en utilisant un gradient discontinu pour obtenir la protéine fusionnée attendue.

On concentre la solution ainsi obtenue sur membrane Amicon à seuil de coupure de 3000 daltons pour obtenir une concentration finale de 10 mg/ml. On obtient ainsi un éluat brut renfermant la protéine recombinante fusionnée de VIH-1 Tat attendue.

On a produit ainsi une protéine de fusion Tat fusionnée génétiquement avec un fragment peptidique riche en histidines qui permet son accrochage au nickel en vue d'une purification.

L'éluat ainsi obtenu sera soumis à la réaction de carboxyméthylation.

### Préparation du toxoïde Tat

### Stade A : Carboxyméthylation

On ajuste une solution de la protéine Tat fusionnée à un fragment peptidique riche en histidine préparée à la préparation 1 ci-dessus pour obtenir les concentrations suivantes: Urée 8M, Tris HCl 0,3M, Ditriothréitol, 10 mM, pH 8,4.

Sous atmosphère inerte, on ajoute 2,6 g d'acide iodoacétique à 100 ml de la solution ci-dessus. Cette solution est incubée à 37°C à l'abri de la lumière et sous atmosphère inerte pendant 90 mn. La réaction est bloquée par addition de 1 ml de β-mercaptoéthanol à 98% et l'incubation est poursuivie pendant 60 minutes dans les mêmes conditions que ci-dessus.

La solution résultant de l'étape précédente est concentrée à l'aide d'un concentrateur Amicon (Cat#8400) sur membrane YM3 (seuil de 3000 D) jusqu'à une concentration de 10 mg/ml.

On la désale alors par passage dans une colonne Cellufine GH25 (MATREX) équilibrée à l'aide de 300 ml d'une solution d'urée 4M, 0,1 M HCl.

### Stade B -Purifications

L'éluat est alors ultrafiltré et traité au bromure de cyanogène pour cliver au niveau de la méthionine la partie amino terminale du produit carboxyméthylé. Du bromure de cyanogène est ajouté en excès sous atmosphère inerte, dans la proportion d'environ 50 moles de bromure de cyanogène par mole de méthionine. Cette solution est conservée dans un récipient clos pendant 24h à 37°C. L'excès de bromure de cyanogène est chassé par évaporation sous pression réduite.

La solution obtenue est alors concentrée, diafiltrée contre du tampon acide acétique-acétate de sodium 0,05M, pH 5 en utilisant un diafiltreur Amicon muni d'une membrane YM3 (seuil 3000 KD). La quantité de produit inactif obtenu en solution est de l'ordre de 450 mg.

Cette solution est filtrée sur une colonne échangeuse d'ions SP-Sépharose FF préalablement équilibrée avec 200 ml de tampon A et le produit est élué par un gradient de NaCl.

L'éluat est à nouveau concentré par diafiltration dans le même système Amicon, et la fraction obtenue est purifiée par gel filtration sur colonne de Sephacryl S (Pharmacia) équilibrée avec du tampon phosphate pH 7,4. Le produit final est filtré sur membrane à 0,22 µm et stocké à 4° jusqu'à utilisation.

Ce produit a été soumis aux analyses ci-dessous, et a fait l'objet des tests pharmacologiques.

### Analyses.

- Quantité totale de produit inactivé dosée par le test de Bradford : 150 mg.
- Electrophorèse sur gel de polyacrylamide SulfoDodécylSulfate-PAGE, coloration argent (Phast System, Pharmacia, gel homogène à 20%) : bande unique avec un poids moléculaire compatible avec celui attendu. Pas d'autres bandes détectables à des poids supérieurs ou inférieurs.
- Electrophorèse par gradient isoélectrique (Phast System, Pharmacia, GEL IEF 3-9): une seule bande visible.
- Western Blot (Phast System, Pharmacia, anticorps monoclonal de l'hybridome 5G11) : une seule bande observée et absence de matériel agrégé ou dégradé.
- Activité biologique (induction du gène CAT dans la lignée HELA-HIV-1-LTR CAT): aucune activité détectable.
- Séquençage de la partie N-terminale en utilisant la technique standard de séquençage d'Edman avec un séquenceur Applied Biosystems (model 477A): les 20 premiers acides aminés obtenus coïncident avec la séquence attendue

   E-P-V-D-P-R-L-E-P-W-K-H-P-G-S-Q-P-K-T-A
- Endotoxines (test LAL selon le protocole USP XXIII): moins de 0,5 unités endotoxique par mg de protéine.
- Stérilité (selon le protocole USP XXIII): répond aux normes.
- Mesure du degré de substitution : en utilisant le réactif d'Ellman pour déterminer les groupements sulfhydriles libres par comparaison avec une courbe standard mesurant les cystéines. Comparant le Tat natif au Tat carboxyméthylé, on a trouvé que seulement 0,03 % des cystéines restaient actives dans le Tat carboxyméthylé, soit 1 cystéine active pour 3330 cystéines inactives. Un simple calcul montre que pour avoir une molécule Tat avec 7 cystéines actives dans ces conditions, il faudrait plusieurs kg de protéine Tat. L'inactivation est donc quasi-totale.

Cette méthode conduit à la production de Tat non-immunosuppresseur, appelé Tat-toxoïde, comme on le verra ci-après.

### Exemple 2. Préparation de Tat mutant obtenu par recombinaison génétique inactif mais immunogène.

Le mutant (AA25 ; Cys versus gly) est obtenu soit par mutagénèse en utilisant le phage M13 soit par PCR. Ce mutant, contrairement à la protéine Tat sauvage, s'est révélé inactif à la dose de 1 µg par ml dans le Cat assay et dans le test d'immunosuppression, de cellules mononuclées du sang périphérique stimulés par des antigènes de rappel (PPD ou Tétanos Toxoïde).

### Exemple 3. Préparation de la Protéine Tat dans un conditionnement huileux la rendant biologiquement inactive.

4 ml d'huile (adjuvant Incomplet de Freund ou ISA 51) sont pipettés dans un flacon refroidi dans un bain de glace. On introduit dans l'huile la tige d'un homogénéiseur, type Silverston, en évitant de toucher les parois du flacon. On ajoute alors à l'huile 4 ml d'immunogène en solution aqueuse , par fraction de 20 µl pour le 1^{er} ml, de 250 µl pour le 3^{ème} ml et de 500 µl pour le 4^{ème} ml. Entre chaque addition, le Silverston est activé à 8000 tours par minute pendant 4 à 5 secondes et après l'addition de toute la solution aqueuse pendant 6 minute à 8000 tours par min. On obtient ainsi une émulsion eau dans l'huile stable. Dans ce conditionnement, la protéine Tat, bien que toujours immunogène, s'est montrée biologiquement inactive. *In vivo*, l'administration par voie sous cutanée d'une émulsion contenant 100 µg de Tat n'a exercé d'effets nocifs ni systémiques (courbe de poids ; température) ni sur le système immunitaire (Réponse cellulaire proliférative normale des splénocytes stimulés par des antigènes de rappel). *In vitro*, la protéine Tat contenue dans la phase aqueuse isolée de l'émulsion s'est montrée antigénique par Test ELISA mais fonctionnellement inactive dans le test du Cat assay.

### Exemple 4. Production de protéine E7 de HPV (souche 16) par les cellules de lignée SIHA dont le génome contient par cellule une copie d'ADN codant cette protéine.

Les cellules de lignée SIHA cultivées dans le milieu MEM contenant 10 % de FCS qui sont concentrées à 5 x 10⁶/ml et incubées avec ou sans sérum à 37°C en présence de 5 % de CO₂ pendant 18 heures. Au terme de cette incubation, la viabilité des cellules est supérieure à 85 %. Sur frottis après coloration au giemsa, ces cellules sont morphologiquement normales. Par référence aux cellules avant incubation, leur cytoplasme apparaît plus basophile, le noyau héberge un ou plusieurs gros nucléoles et les mitoses sont moins nombreuses ou inexistantes. Ces cellules exportent dans le surnageant de culture des quantités de protéine E7 mesurables et identifiées spécifiquement par test d'immunoblot utilisant des anticorps anti-E7 de lapin révélés par ELISA. Par référence aux immuno-empreintes faites sur des quantités connues décroissantes de protéine E7 recombinante purifiée, ces cellules en culture ont produit dans le milieu extracellulaire des quantités de E7 supérieures à 10 pg/ml.

### Exemple 5. Fabrication de Protéine E7 inactivée par traitement chimique.

Des essais utilisant le glutaraldéhyde ont permis de produire une protéine E7 inactivée dans les tests d'immunosuppression. Le traitement par le glutaraldéhyde s'effectue de la façon suivante: une solution d'E7 à 1 mg/ml en phosphate disodique 70 mM à pH 8,2 est additionnée de glutaraldéhyde à concentration finale 0,026M ou 0,0026M. On laisse la réaction s'accomplir durant des temps variables allant de 1 mn à 3 heures à la température ambiante. A la fin de chaque temps de réaction, la réaction est bloquée par addition de glycine à concentration finale 100 mM. Les différents prélèvements sont dialysés pendant 16h, à 4°C, contre 100 fois leur volume de PBS. La dialyse est répétée 2 fois. On constate dans tous les cas que dès après 1 heure de traitement par le glutaraldéhyde, l'effet immunosuppressif d'E7 a disparu ainsi que mesuré dans le test d'immunosuppression. Il convient d'optimiser les conditions de la réaction afin de préserver au maximum l'immunogénicité de la protéine: ceci se fait en testant les préparations selon la méthode utilisée dans l'expérimentation 3.

Des essais ont aussi été réalisés avec le formaldéhyde. à la concentration finale de 33 mM, et l'inactivation est testée de 1 heure à plusieurs jours. La réaction est bloquée par addition de glycine, avant d'être dialysée de façon répétée contre du PBS. La protéine obtenue, ou E7-toxoïde, contrairement à la protéine native, n'a pas montré d'activité immunosuppressive dans le test d'activité des cellules mononuclées du sang périphérique activées par les antigènes de rappel (PPD et Tétanos Toxoïde).

### Exemple 6. Fabrication de protéine Tax inactivée par traitement chimique.

La protéine Tax a été produite par génie génétique et obtenue à la concentration de 1 mg/ml. L'application du même protocole d'inactivation, utilisant la carboxamidation, a conduit à l'obtention d'un produit dérivé Tax-toxoïde inactif dans le test d'immunosuppression de cellules mononuclées du sang stimulées par des antigènes de rappel (PPD et Tétanos Toxoïde).

### Exemple 7

On a préparé une composition injectable répondant à la
formule composée de l'exemple 1 50 µg
excipient q.s. pour une ampoule terminée à 0,5 ml
(détail de l'excipient : adjuvant ISA 51 (Seppic))

### Exemple 8

On a préparé une composition injectable répondant à la formule composée de l'exemple 3 100 µg
excipient q.s. pour une ampoule terminée à 1 ml
(détail de l'excipient :hydroxyde d'alumine (Superfos, Kvistgaard, DK))

### Etude pharmacologique

### Expérimentation 1 a. Propriétés immunosuppressives de facteurs protéiques extracellulaires provenant de cellules cancéreuses, de cellules infectées par des virus ou de cellules immunitaires.

1a1. On a purifié la protéine Tat du VIH-1 à la concentration de 1 mg/ml comme préparé par Frankel A.D. et al. (Cellular uptake of the tat protein from human immunodeficiency virus, Cell, 1988, 55 : 1189-93).

On prend des cellules mononucléées du sang périphérique (PBMCs) d'un sujet sain purifiées par Ficoll que l'on met en culture à 1 millions de cellules par ml dans des micropuits en présence d'antigènes de rappel PPD (Mérieux, dilution au 1/1000) dans du RPMI, sérum SAB 10%. On ajoute de la protéine Tat à dose décroissante: 10 mg/ml, 3 mg/ml, 1 mg/ml etc... Pour obtenir des résultats plus marqués, il est parfois nécessaire de préincuber les cellules 2h dans le RPMI avec la protéine Tat avant de rajouter le sérum, ceci parce que la protéine peut parfois se lier à des composants du sérum et ainsi inactiver sa capacité d'agir sur les cellules.

On ajoute de l'IL2 après 48h. Enfin après 5 jours, on mesure dans les puits la prolifération cellulaire par le test d'incorporation de thymidine tritiée. Les résultats sont les suivants:

| Prolifération des PBMCs (CPM) | |
|---|---|
| Sans addition de Tat | 28 000 |
| Tat (10 µg/ml) | 8 000 |
| Tat (3 µg/ml) | 13 000 |
| Tat (1 µg/ml) | 21 000 |
| Tat (0,3 µg/ml) | 26 000 |

1 a2. Cytokines immunosuppressives couvertes par le Brevet international WO 92/225.

1a3. Lectines immunosuppressives. Nous avons récemment identifié une lectine produite par les lymphocytes T, dont l'haptène est le mannane et qui est immunosuppressive. L'inhibition de la réponse immunitaire cellulaire est mise en évidence 1) par l'augmentation de la production d'IFNα, cytokine immunosuppressive, par les APC. Cette augmentation est bloquée en présence de mannane et non de lactose ou de fructose ; 2) par l'inhibition de la prolifération des lymphocytes T, PBMC stimulées par des antigènes de rappel (PPD, Tétanos Toxoïde) ou par des alloantigènes.

Expérimentalement, cette lectine a été mise en évidence dans les surnageants de cellules H9 cultivées sur milieu HL1 sans sérum, ou de PBMC préactivées par la cytokine IL-2 et cultivées sur milieu HL1 sans sérum. Les surnageants des cultures ont été concentrés par diafiltration sur membrane avec coupure a 10 KDa. Ces surnageants concentrés présentent les propriétés suivantes :
1) d'agglutiner les cellules H9 inactivées par la mytomicine ; laquelle agglutination est empêchée par le mannane.
2) de stimuler, à faible concentration, la prolifération des cellules H9.
3) d'inhiber, à forte concentration, la prolifération des cellules H9

| Agglutination de cellules fixées par des surnageants de H 9 ou PBMCs | | | | | | | |
|---|---|---|---|---|---|---|---|
| | Dilutions des surnageants | | | | | | |
| | 1/2 | 1/4 | 1/8 | 1/16 | 1/32 | 1/64 | 1/28 |
| HL1 médium > 30kDa | 0* | 0 | 0 | 0 | 0 | 0 | 0 |
| HL1 médium < 30kDa | 0 | 0 | 0 | 0 | 0 | 0 | 0 |
| H9 SN > 30kDa | 4 | 4 | 3 | 2 | 2 | 1 | 0 |
| H9 SN < 30kDa | 0 | 0 | 0 | 0 | 0 | 0 | 0 |
| PBMC SN > 30kDa | 4 | 3 | 2 | 1 | 0 | 0 | 0 |
| PBMC SN < 30kDa | 0 | 0 | 0 | 0 | 0 | 0 | 0 |

| | | | | | | | |
|---|---|---|---|---|---|---|---|
| * Scores d'agglutination | | | | | | | |

| Prolifération des cellules H9 en présence de surnageants riches en lectine | | | | | |
|---|---|---|---|---|---|
| | Dilutions des surnageants | | | | |
| | 0 | 1/2 | 1/4 | 1/8 | 1/16 |
| HP SN > 30kDa | 12 000* | 8 000 | 16 000 | 21 000 | 13 500 |
| HP SN < 30kDa | 12 000 | 12500 | 11 800 | 12 000 | 12500 |

| | | | | | |
|---|---|---|---|---|---|
| * Mesure par test d'incorporation de 3H Thymidine (cpm). | | | | | |

Ces propriétés permettent d'identifier le facteur présent dans les surnageants à une lectine.

Les propriétés immunosuppressives de cette lectine ont pu être rapportées au fait que l'action de ces surnageants sur les APC (cellules dendritiques, macrophages) en culture activées par l'IFNγ s'objective par une forte augmentation de la production d'IFNα par ces cellules. Cependant, parallèlement à l'augmentation de la production d'IFNα, on observe la perte de pouvoir antiviral de l'IFNα sur les VSV lorsque les cellules MDBK sont prétraitées par les surnageants de culture.

| Surnageant de culture | Neutralisation sur MDBK (%) |
|---|---|
| milieu contrôle | 100 |
| H9 | 50 |
| H9 mannane | 100 |
| PBMC | 40 |
| PBMC mannane | 80 |

| Surnageant de culture | Augmentation de la production d'IFNα par les APC activés (%) |
|---|---|
| milieu contrôle | 100 |
| H9 | 250 |
| PBMC | 300 |

### 1a4. Cas de la protéine E7 du papilloma virus.

On a réalisé la même expérience qu'avec Tat avec de la protéine E7 du papilloma virus purifiée selon Speidel et al, Eur. J. Immunol., 1997, 27, pages 2391-2399.

Les tests d'incorporation de la 3H-Thymidine a donné des résultats similaires.

| Prolifération des PBMCs (CPM) | |
|---|---|
| Sans addition de CE7 | 29 000 |
| E7 (10µg/ml) | 6 000 |
| E7 (3 µg/ml) | 10 000 |
| E7 (1 µg/ml) | 11 000 |
| E7 (0,3 µg/ml) | 16 000 |

### 1a5. Cas de la protéine Tax de HTLV-1.

On a aussi réalisé la même expérience qu'avec Tat avec de la protéine Tax de HTLV-1 purifiée obtenue de Tanaka et al. Int. J. Cancer, 1991, vol 48, pages 623-630.

Les tests d'incorporation de la 3H-Thymidine a donné des résultats similaires.

| Prolifération des PBMCs (CPM) | |
|---|---|
| Sans addition de Tax | 25 000 |
| Tax (10 µg/ml) | 7 000 |
| Tax (3 µg/ml) | 15 000 |
| Tax (1 µg/ml) | 21 000 |
| Tax (0,3 µg/ml) | 26 000 |

Ces résultats montrent que les protéines natives ci-dessus sont capables d'induire une immunosuppression dose-dépendante et ont la capacité *de démobiliser le système immunitaire. Propriétés angiogéniques de la protéine* E7.

### Expérimentation 2. Propriétés angiogéniques de facteurs extracellulaires protéiques.

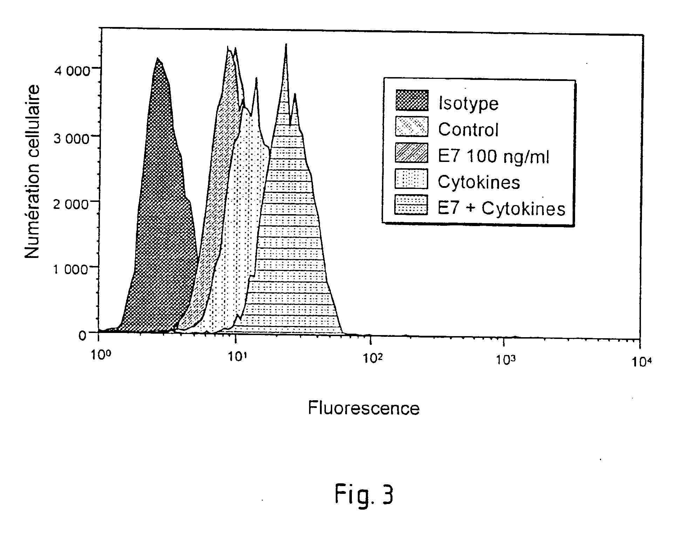

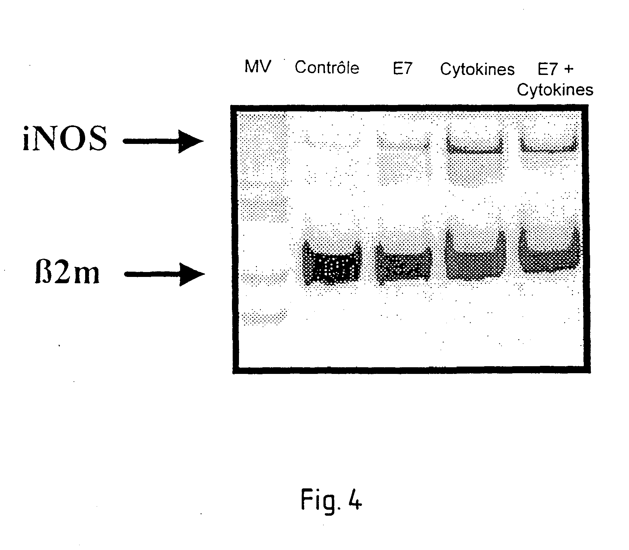

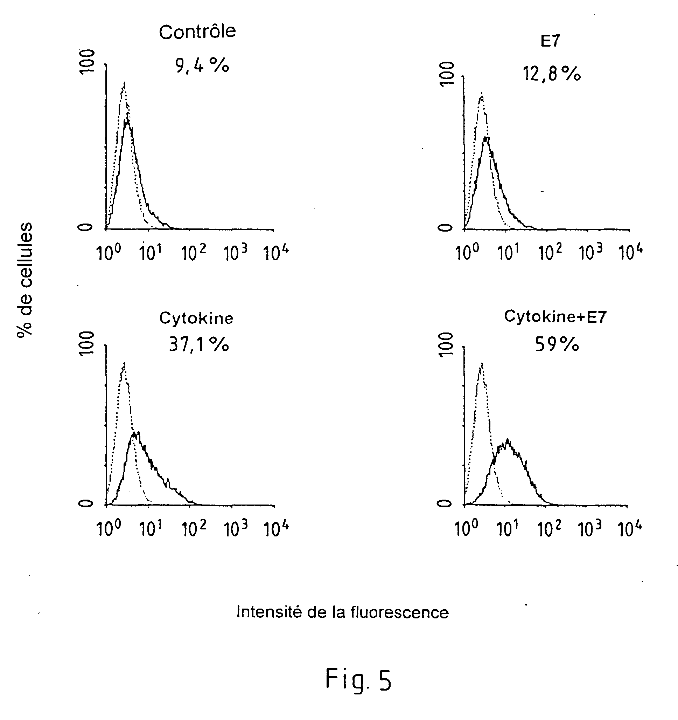

La protéine E7 de l'HPV (souche 16) purifiée qui augmente significativement la production des cytokines TNFα et IL-1 par les macrophages et les cellules dendritiques a également montré des propriétés angiogéniques au sein de cultures de cellules endothéliales provenant de cordon ombilical humain (HUVEC). En effet, nous avons observé que l'action de la protéine E7 sur ces cellules cultivées dans un milieu renfermant de l'EGF et stimulées ou non par les cytokines, TNFα ; IFNγ ou IL-1β se traduisait par l'augmentation significative du taux de production des NO synthases, comme cela a été établi par FACS en cytométrie de fluorescence (Figure 3) ou par PCR après extraction du RNA total des cellules traitées par la protéine E7 ou les cytokines (Figure 4). On a également montré que la protéine E7, soit seule, soit en association avec des cytokines, augmentait significativement l'expression par ces mêmes cellules des molécules d'adhésion ICAM-1 (Figure 5).

### Expérimentation 3. Absence d'activité suppressive des toxoïdes dans le test d'immunosuppression.

On a testé dans les mêmes conditions que celles décrites dans l'expérimentation 1, les protéines modifiées par traitement chimique des exemples 1, 2 et 3.

On n'a pas pu mettre en évidence d'effet immunosuppressif pour ces produits:

| Prolifération des PBMCs (% par rapport au contrôle) | |
|---|---|
| Contrôle | 100 |
| Tat Inactivé(10µg/ml) | 95 |
| Tat natif (10µg/ml) | 20 |
| E7 Inactivée | 105 |
| E7 natif | 25 |
| Tax Inactivé | 100 |
| Tax natif | 25 |

### Expérimentation 4. Immunogénicité des protéines des exemples 1, 2 et 3

Des toxoïdes préparés selon les exemples 1, 2 et 3 ont été utilisés pour immuniser des souris.

Le protocole d'immunisation est celui classiquement utilisé: les souris sont injectées (en im) avec 100 µl d'émulsion (1:1) en adjuvant complet de Freund contenant 20 µg de produit aux jour 0 avec rappel en adjuvant incomplet de Freund de 5 µg aux jours 21 et 35. Le sérum des souris est prélevé aux jours -2 et 40 et analysé par ELISA sur des plaques sensibilisées avec la protéine native correspondante (non traitée chimiquement) et avec les toxoïdes préparés aux exemples 1, 2 et 3. Les sérums ont été testés à une dilution au 1/500.

Les résultats obtenus ont été comparables pour les 3 produits testés ici, Tat-toxoïde, Tax-toxoïde et E7-toxoïde.

Ces résultats, exprimés en densité optique, obtenus sur 3 souris immunisées (1 à 3) et 3 souris non immunisées (4 à 6), sont rapportés dans le tableau suivant:

| | Protéine native | Protéine Inactivée |
|---|---|---|
| Souris 1 | | |
| J-2 | 0,2 | 0,2 |
| J40 | 2,1 | 2,1 |
| Souris 2 | | |
| J-2 | 0,15 | 0,2 |
| J40 | 2,2 | 2,2 |
| Souris 3 | | |
| J-2 | 0,2 | 0,1 |
| J40 | 1,9 | 2,1 |
| Souris 4 | | |
| J-2 | 0,15 | 0,15 |
| J40 | 0,1 | 0,1 |
| Souris 5 | | |
| J-2 | 0,1 | 0,1 |
| J50 | 0,1 | 0,15 |
| Souris 6 | | |
| J-2 | 0,2 | 0,2 |
| J40 | 0,1 | 0,1 |

Ces résultats montrent que les souris immunisées par le toxoïde produisent des anticorps capables de reconnaître de la même façon la protéine native et le toxoïde. D'autre part cela confirme l'innocuité de l'immunisation par les toxoïdes car les souris ont très bien toléré l'immunisation.

### Expérimentation 5. Immunisation chez l'homme par le produit de l'exemple 1.

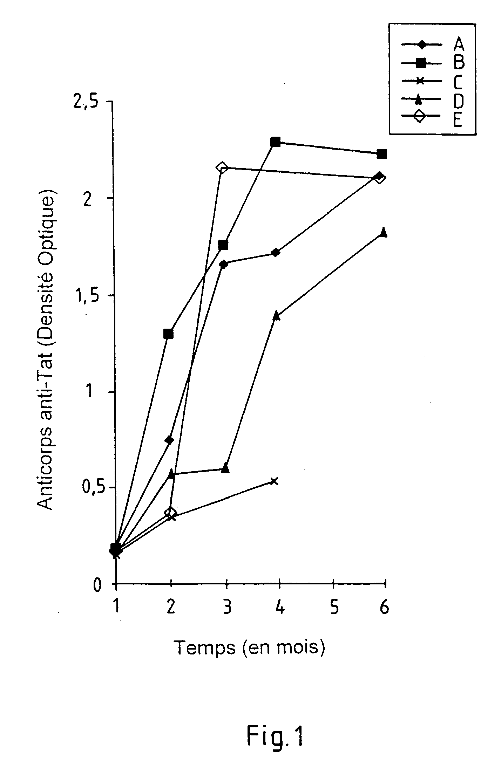

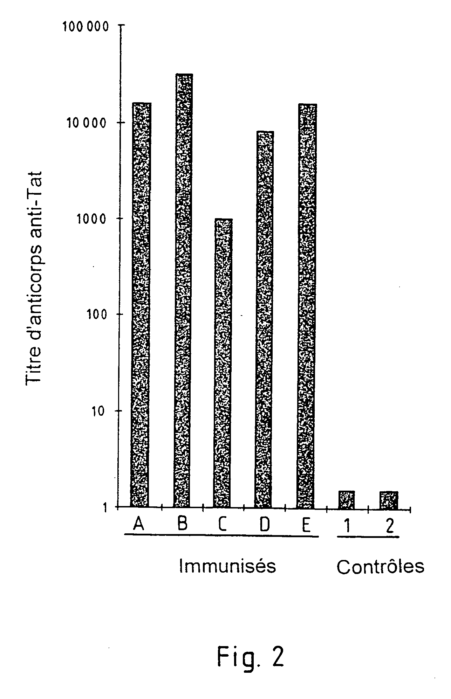

5 sujets séronégatifs ont subi une immunisation avec la protéine Tat inactivée immunogène de l'exemple 1 dans de l'adjuvant incomplet de Freund (Isa 51, Seppic) suivie d'un premier rappel environ 3 semaines après et d'un deuxième rappel environ 15 jours après. Une réponse immunitaire anti protéine Tat native a pu être objectivée par tests ELISA. La Figure 1 présente les réponses anti-Tat des 5 sujets immunisés, déterminées par ELISA sur plaques cotées par la protéine Tat native, après l'immunisation au Tat toxoïde chez les 5 sujets et les différents rappels. La figure 2 présente les titres obtenus après toutes les immunisations, et ceci comparé à 2 contrôles non immunisés.

On voit donc que le toxoïde peut être utilisé chez l'homme et provoque une réponse immunitaire qui produit une réaction croisée avec la protéine Tat native.

## Revendications

1. Composé immunogène **caractérisé en ce qu'**il est une protéine initialement immunosuppressive et/ou angiogénique choisie parmi :
- la protéine Tat du virus HIV-1 ;
- la protéine Tax d'un virus HTVL-1 ; et
- la protéine E7 du Papillomavirus;
ou encore un fragment comprenant au moins 50% de la totalité de cette protéine, et **en ce que** cette protéine est inactivée par traitement chimique par carboxamidation ou maléimidation.

2. Composé immunogène selon la revendication.1, **caractérisé en ce que** cette protéine est inactivée par traitement chimique par carboxamidation.

3. Composé immunogène selon la revendication 1, **caractérisé en ce que** cette protéine est inactivée par traitement chimique par maléimidation.

4. Un composé immunogène tel que défini dans l'une des revendications 4 à 6, pour son utilisation dans une méthode de traitement du corps humain
ou animal.

5. Procédé préparation d'un composé tel que défini dans l'une des revendications 1 à 4, **caractérisé en ce que** l'on soumet une protéine initialement immunosuppressive ou angiogénique choisie parmi :
- la protéine Tat du virus HIV-1 ;
- la protéine Tax d'un virus HTVL-1 ; et
- la protéine E7 du Papillomavirus ;
ou encore un fragment comprenant au moins 50% de la totalité de cette protéine à un traitement chimique par carboxamidation ou maléimidation.

6. Composition vaccinale comprenant un composé immunogène tel que défini dans l'une des revendications 1 à 4.

## Claims

1. Immunogenic compound **characterized in that** it is an initially immunosuppressive and/or angiogenic protein selected from:
- the protein Tat of HIV-1 virus
- the protein Tax of HTVL-1 virus, and
- the protein E7 of papillomavirus
or a fragment comprising at least 50% of the entirety of the said protein, and that the said protein is inactivated by chemical treatment by carboxamidation or maleimidation.

2. Immunogenic compound according to claim 1, **characterized in that** the protein is inactivated by chemical treatment by carboxamidation.

3. Immunogenic compound according to claim 1, **characterized in that** the protein is inactivated by chemical treatment by maleimidation.

4. An immunogenic compound as defined in anyone of claims 4 to 6, for use in a method for treating the human or animal body.

5. Method for preparing a compound as defined in anyone of claims 1 to 4, **characterized in that** an initially immunosuppressive and/or angiogenic protein selected from
- the protein Tat of HIV-1 virus
- the protein Tax of HTVL-1 virus, and
- the protein E7 of papillomavirus
or a fragment comprising at least 50% of the entirety of the said protein is subjected to a chemical treatment by carboxamidation or maleimidation.

6. Vaccine composition comprising an immunogenic compound as defined in one of claims 1 to 4.

## Patentansprüche

1. Immunogene Verbindung, **dadurch gekennzeichnet, dass** es sich um ein anfangs immunsuppressives und/oder angiogenes Protein handelt, das ausgewählt ist aus:
- dem Tat-Protein des Virus HIV-1;
- dem Tax-Protein eines HTLV-1-Virus; und
- dem E7-Protein des Papillomvirus;
oder auch um ein Fragment, das wenigstens 50% der Gesamtheit dieses Proteins umfasst, und **dadurch**, dass dieses Protein durch chemische Behandlung mittels Carboxamidierung oder Maleinimidierung inaktiviert ist.

2. Immunogene Verbindung gemäß Anspruch 1, **dadurch gekennzeichnet, dass** dieses Protein durch chemische Behandlung mittels Carboxamidierung inaktiviert ist.

3. Immunogene Verbindung gemäß Anspruch 1, **dadurch gekennzeichnet, dass** dieses Protein durch chemische Behandlung mittels Maleinimidierung inaktiviert ist.

4. Immunogene Verbindung, wie sie in einem der Ansprüche 4 bis 6 definiert ist, zur Verwendung in einem Verfahren zur Behandlung des menschlichen oder tierischen Körpers.

5. Verfahren zur Herstellung einer Verbindung, wie sie in einem der Ansprüche 1 bis 4 definiert ist, **dadurch gekennzeichnet, dass** ein anfangs immunsuppressives und/oder angiogenes Protein, das ausgewählt ist aus:
- dem Tat-Protein des Virus HIV-1;
- dem Tax-Protein eines HTLV-1-Virus; und
- dem E7-Protein des Papillomvirus;
oder auch ein Fragment, das wenigstens 50% der Gesamtheit dieses Proteins umfasst, einer chemischen Behandlung mittels Carboxamidierung oder Maleinimidierung unterzogen wird.

6. Impfzusammensetzung, die eine immunogene Verbindung, wie sie in einem der Ansprüche 1 bis 4 definiert ist, umfasst.
